# EUROPÄISCHE PATENTSCHRIFT

(11) **EP 3 516 037 B1**
(45) Veröffentlichungstag und Bekanntmachung des Hinweises auf die Patenterteilung: **19.02.2020**
(21) Anmeldenummer: 17780646.0
(22) Anmeldetag: 19.09.2017
(51) Int. Cl.: C12M 1/107, C12M 1/00

(54) **BEHÄLTER UND BIOGASANLAGE**
CONTAINER AND BIOGAS INSTALLATION
RÉSERVOIR ET INSTALLATION DE BIOGAZ

(30) Priorität: 20.09.2016 DE 102016218051
(43) Veröffentlichungstag der Anmeldung: 31.07.2019
(73) Patentinhaber: bioenergy concept GmbH, 21335 Lüneburg (DE)
(72) Erfinder: EUSTERBROCK, Christoph, 21407 Deutsch Evern (DE)
(74) Vertreter: Knoop, Philipp
(86) Internationale Anmeldenummer: PCT/EP2017/073674
(87) Internationale Veröffentlichungsnummer: WO 2018/054920

(56) Entgegenhaltungen:
- DE-A1- 2 407 759
- DE-A1- 10 223 866
- DE-A1- 19 719 323
- DE-A1-102008 038 262

## Beschreibung

Die vorliegende Erfindung betrifft einen Behälter, insbesondere zur Erzeugung von Biogas, mit einem, vorzugsweise zylindrischen, Hauptabschnitt und einem unterhalb des Hauptabschnitts angeordneten Bodenabschnitt.

Die Erfindung betrifft ferner eine Biogasanlage.

Behälter der eingangs genannten Art sind beispielsweise als Bestandteil von Biogasanlagen in unterschiedlichen Ausgestaltungen und unterschiedlichen Größen bekannt. Häufig ist der Behälter als Stahltank oder Stahlbetontank ausgestaltet und weist ein Volumen von 400-6000 m³ auf. In den Behälter wird in Form von flüssigem Schlamm die zur Biogaserzeugung vorgesehene Biomasse eingefüllt. Der flüssige Schlamm ist dabei eine Suspension, so dass das Problem von Ablagerungen besteht. Bei herkömmlichen Behältern von Biogasanlagen ist der Boden im Wesentlichen eben ausgeformt. In der Praxis ist es bei den vorbekannten Behältern daher häufig ein Problem in Biogasanlagen, dass Verstopfungen und eine Ablagerung von Schwerstoffen am Boden erfolgt, aufgrund des Absackens der schweren Bestandteile des zu behandelnden flüssigen Schlamms.

Um diese Thematik anzusprechen, ist aus der DE 42 08 148 A1 eine Vorrichtung zur anaeroben Zusetzung von Schlamm vorbekannt, die in einem unteren Gehäusebereich konisch ausgestaltet ist bzw. eine Eiform aufweist. Eine wichtige Wirkung des konischen oder eiförmigen Bodenkegels besteht gemäß dem Stand der Technik darin, eine Übergangszone zur Verfügung zu stellen, um in dem Faulbehälter enthaltene Zersetzungsflüssigkeit zu oder aus dem Gehäuseboden und dem unteren Ende des Saugrohres zu bewegen. Daher soll gemäß dem Stand der Technik die Neigung des konischen Gehäuses gewählt sein, um sicherzustellen, dass sich der zersetzende Schlamm nicht ablagert und so von dem Mischprozess isoliert wird. Nachteilig an dem aus der DE 4 208 148 A1 vorbekannten Behälter ist jedoch, dass das Problem von Verstopfungen und Trichterbildungen aufgrund von Schlammablagerungen in der Praxis fortbesteht bzw. dass die Investitionskosten bei dieser Bauform sehr hoch sind.

In der Regel werden daher bei herkömmlichen Behältern zur Erzeugung von Biogas großdimensionierte Rührwerke eingesetzt, um Verstopfungen und Sedimentierungen wirksam zu unterbinden. Mit Nachteil sind der apparative Aufwand, die Investitionskosten sowie die Betriebskosten aufgrund des hohen Energieaufwands jedoch beträchtlich.

Aus der DE 102 23 866 A1 ist ein Gärbehälter mit einem in einem Innenraum angeordneten Rührwerk und mit mindestens einer dem unteren Behälterbereich zugeordneten Austragsöffnung für Sedimente, der zumindest ein bewegliches Förderelement für die Sedimente zugeordnet ist, bekannt, wobei als Zuführung in die Austragsöffnung eine Ringleitung vorgesehen ist, die sich im radial äußeren Bereich des Innenraums erstreckt.

Die DE 10 2008 038 262 A1 macht ebenfalls ein Verfahren und eine Vorrichtung zur Vergärung von Biomasse vorbekannt, wobei ein Gärrest über eine Rohrleitung abgelassen wird.

Die DE 197 19 323 A1 offenbart ein Tunnelfermentationsverfahren zur einstufigen anaeroben und aeroben Behandlung von festen und flüssigen biogenen Abfällen, wobei ein Tunnelfermenter doppelwandig ausgeführt ist und die flüssigen oder pastösen Substrate mittels einer Schlammwasserpumpe abziehbar und dem Verfahren gezielt wieder zuführbar sind.

Die DE 24 07 759 A1 offenbart einen flexiblen Faulturmbehälter.

Vor dem geschilderten Hintergrund liegt der vorliegenden Erfindung die Aufgabe zu Grunde, einen Behälter der eingangs genannten Art und eine Biogasanlage anzugeben, welche einen einfachen, kostengünstigen Aufbau haben, der eine einfache Wartung erlaubt, und bei geringem Energieverbrauch wirksam Verstopfungen und eine Sedimentierung am Behälterboden vermeidet.

Erfindungsgemäß wird die auf den Behälter gerichtete Aufgabe durch einen Behälter mit der Merkmalskombination des Anspruchs 1, insbesondere zur Erzeugung von Biogas, mit einem, vorzugsweise zylindrischem, Hauptabschnitt und einem unterhalb des Hauptabschnitts angeordneten Bodenabschnitt gelöst, bei dem der Bodenabschnitt einen ringförmig geschlossenen Zirkulationskanal aufweist. Indem am Boden des Behälters ein Ringkanal zum Führen eines Zirkulationsstroms um die Hochachse des Behälters angeordnet ist, können mit Vorteil zu Verstopfung und Sedimentierung führende Ablagerungen im Bodenbereich des Behälters wirksam vermieden werden. Mit Vorteil kann ein Rührwerk im Hauptabschnitt des Behälters auf diese Weise erheblich kleiner dimensioniert werden oder im günstigsten Falle vollständig entfallen, ohne dass Verstopfungen zu besorgen sind. Mit Vorteil können sowohl der Energieverbrauch der Anlage als auch anderweitige Betriebskosten gegenüber herkömmlichen Behältern verringert werden.

Bevorzugt weist in Ausgestaltung der Erfindung der Bodenabschnitt einen im Wesentlichen ebenen Zentralbereich auf, welcher horizontal von dem Zirkulationskanal begrenzt ist. Mit Vorteil ist somit ein zentraler ebener Bodenabschnitt von einem Zirkulationskanal umgeben. Der Zirkulationskanal kann dabei beispielsweise als ringförmig geschlossene Rinne ausgestaltet sein, deren Boden tiefer als der ebene Zentralbereich des Bodenabschnitts liegt. Durch die Zirkulation in dem Zirkulationskanal werden erfindungsgemäß mit Vorteil Ablagerungen in dem ebenen Zentralbereich unterbunden, wenn man im Zirkulationskanal mit geeigneten Mitteln eine Zirkulation erzeugt.

Insbesondere weist bei dem erfindungsgemäßen Behälters der Zirkulationskanal eine Einlassöffnung mit einem Einlassquerschnitt auf, um im Wesentlichen tangential zur Hochachse des Behälters Medium in den Zirkulationskanal einzulassen. Auf diese Weise kann durch die Zufuhr von Medium durch die Einlassöffnung eine Zirkulation im Zirkulationskanal erzeugt werden, welche mit Vorteil Ablagerung von Feststoffen im Bodenbereich entgegenwirkt.

Ebenfalls vorteilhaft ist es, dass bei dem erfindungsgemäßen Behälter der Zirkulationskanal eine Auslassöffnung mit einem Auslassquerschnitt aufweist, um im Wesentlichen tangential zur Hochachse des Behälters Medium aus den Zirkulationskanal abzuführen. Beispielsweise kann an die Auslassöffnung eine Pumpe angeschlossen werden, um Medium entlang des Zirkulationskanals aus dem Behälter abzusaugen. Dadurch entsteht im Zirkulationskanal eine ringförmige Zirkulation, welche mit Vorteil Ablagerungen im Bodenbereich unterbinden kann. Mit Vorteil ist es zur Erreichung dieser Wirkung nicht erforderlich, im Hauptabschnitt des Behälters energieintensive Rührwerke vorzusehen deren Rührleistung ausreicht, um auch im Bodenbereich Umwälzungen hervorzurufen. Ein eventuell vorgesehenes Rührwerk im Hauptabschnitt kann nach der Erfindung daher erheblich kleiner ausgelegt werden. Der Betrieb einer Biogasanlage mit einem derartigen Behälter kann somit mit Vorteil kostengünstig da energiesparend erfolgen.

Da eine die Auslassöffnung mit der Einlassöffnung verbindende Bypass-Leitung vorgesehen ist, kann im Zirkulationskanal eine zirkulierende Strömung hervorgerufen werden, welche ganz oder teilweise auf einer Rückführung aus dem Behälter abgeführten Mediums basiert. Insbesondere kann ein Teil des über die Auslassöffnung aus dem Zirkulationskanal abgefüllten Mediums einem Lager zugeführt werden für die Lagerung fester Rückstände aus der Biogaserzeugung. Andererseits kann über die Bypass-Leitung ein Teilstrom aus dem abgepumpten Strom abgezweigt und über die Einlassöffnung in den Zirkulationskanal rückgeführt werden.

In vorteilhafter Ausgestaltung des erfindungsgemäßen Behälters ist eine Einlasspumpe zum Einlassen von Medium in den Zirkulationskanal über die Einlassöffnung und/oder eine Auslasspumpe zum Abführen von Medium aus dem Zirkulationskanal über die Auslassöffnung vorgesehen. Mit Vorteil lässt sich auf diese Weise im Rahmen der Erfindung im Zirkulationskanal durch Einsatz einer Pumpe eine Zirkulation aufbauen. Wenn eine separate Einlasspumpe und eine separate Auslasspumpe vorgesehen sind, kann durch Steuerung der Leistung der Einlasspumpe bzw. der Auslasspumpe relativ zueinander das Verhältnis zwischen rückgeführtem Medium und abgeführtem Medium eingestellt werden. Andererseits kann im Rahmen der Erfindung auch eine einzige Pumpe eingesetzt werden, sofern eine Bypass-Leitung vorgesehen ist, welche die Auslassöffnung mit der Einlassöffnung verbindet.

In diesem Zusammenhang ist es günstig, dass Mittel zum Variieren eines Querschnitts der Bypass-Leitung vorgesehen sind. Beispielsweise können erfindungsgemäß die Mittel zum Variieren als Schieber ausgestaltet sein, welcher den Querschnitt der Bypass-Leitung mehr oder minder freigibt. Auf diese Weise kann bei Verwendung einer Pumpe in dem Zirkulationssystem das Verhältnis von zum Lager abgepumpte Medium zu in den Zirkulationskanal rückgeführten Medium kontinuierlich während des Betriebs eingestellt werden.

Um ein Verhältnis zwischen rückgeführtem und abgeführtem Medium einzustellen, ist es im Rahmen der Erfindung günstig, wenn der Auslassquerschnitt größer als der Einlassquerschnitt ausgestaltet ist. In diesem Falle wird eine größere Menge sedimenthaltigen Schlamms aus dem Zirkulationskanal abgeführt und eine kleinere Menge über den Einlass in den Zirkulationskanal rückgeführt, sofern die Einlassöffnung und die Auslassöffnung über eine Bypass-Leitung kommunizieren.

In der Praxis hat es sich als besonders geeignet erwiesen, wenn der Durchmesser der Auslassöffnung etwa zweimal so groß ist wie der Durchmesser der Einlassöffnung.

Als vorteilhaft hat es sich in Ausgestaltung der Erfindung erwiesen, wenn der Zirkulationskanal aus einem Kunststoff hergestellt ist und vorzugsweise mit dem Zentralbereich vergossen ist. Die Herstellung und Ausformung des kreisförmig geschlossenen Zirkulationskanal kann insbesondere unter Verwendung von Polyethylen kostengünstig erfolgen. Der Ringkanal aus Polyethylen kann sodann mit dem beispielsweise als Stahlbetonboden ausgestalteten Zentralbereich vergossen werden.

In Weiterbildung des erfindungsgemäßen Behälters schließt sich in Richtung der Hochachse des Behälters an den Bodenabschnitt ein in Richtung zum Bodenabschnitt, vorzugsweise konisch, verjüngter Abschnitt an. Der zum Beispiel konisch verjüngte Bereich hat dabei erfindungsgemäß insbesondere eine Art Trichterform. Die Trichterform bewirkt mit Vorteil eine Verjüngung, d.h. Verringerung, des Querschnitts des Behälters in Richtung zum Boden. In der Praxis kann der Behälter im Bereich des vorzugsweise zylindrischen Hauptabschnitts zum Beispiel einen Durchmesser von 30 m aufweisen. Demgegenüber hat es sich erfindungsgemäß als günstig erwiesen, wenn der ebene Zentralbereich des Bodenabschnitts einen Durchmesser von lediglich etwa 3 m - 6 m aufweist. Aufgrund des erfindungsgemäßen verjüngten Behälterabschnitts wird der Behälterquerschnitt ausgehend von einem relativ großen Querschnittswert im Bereich des Hauptabschnitts gleichmäßig auf einen viel kleineren Durchmesserwert im Bereich des Zentralbereichs vermindert. Dies ist zum einen vorteilhaft, um aufgrund der Strömungsverhältnisse ein Ablagern von Sediment im Bodenbereich entgegenzuwirken, wenn im Hauptabschnitt ein Rührwerk betrieben wird. Zum anderen hat sich gezeigt, dass die Wirkung eines Zirkulationskanals zur Unterbindung von Ablagerungen im Bodenabschnitt ein ebener Zentralbereich des Bodenabschnitts keinen zu großen Durchmesser haben darf. Der verjüngte Behälterabschnitt, insbesondere konische Abschnitt, wirkt somit gleichsam als Adapter zwischen dem Hauptabschnitt des Behälters und dem Bodenabschnitt.

Dabei kann im Rahmen der Erfindung im Prinzip auch der Hauptabschnitt des erfindungsgemäßen Behälters vorzugsweise konisch verjüngt ausgestaltet sein.

Alternativ ist im Rahmen der Erfindung der konisch verjüngte Behälterabschnitt zwischen dem Bodenabschnitt und dem Hauptabschnitt angeordnet.

Um den, insbesondere konisch, verjüngten Abschnitt nach der Erfindung besonders kostengünstig herstellbar zu gestalten, weist der verjüngte Abschnitt Kunststoffwandungen aus. Als Werkstoff hat sich insbesondere auch unter Berücksichtigung von Grundwasserschutzaspekten PE-HD als geeignet erwiesen. Dieses Material ist beispielsweise bereits bei Ausgestaltungen von Biogasanlagen erprobt, welche als Lagunen, also als mit Folie ausgekleidete Erdbecken, ausgestaltet sind.

Um ein Sedimentieren von Sinkstoffen am Boden des Behälters zusätzlich entgegenzuwirken, können nach der Erfindung in dem Behälter im Hauptabschnitt Rührmittel zum Rühren im Behälter befindlichen Mediums vorgesehen sein. Mit Vorteil können die Rührmittel jedoch aufgrund der erfindungsgemäßen Ausgestaltung des Behälters mit einem ringförmig geschlossenen Zirkulationskanal im Bodenabschnitt erheblich geringer dimensioniert sein. Dadurch wird die Energieaufnahme einer Biogasanlage bei Einsatz eines erfindungsgemäßen Behälters signifikant vermindert.

Die obige Aufgabe wird erfindungsgemäß gleichermaßen durch eine Biogasanlage gelöst, welche einen Behälter nach einem der Ansprüche 1 bis 8 aufweist, der zumindest mit dem Bodenabschnitt in das Erdreich eingelassen ist, wobei unterhalb des Bodenabschnitts ein Kiesbett vorgesehen ist. Anhand des Kiesbetts lassen sich insbesondere unter Umständen bestehende Wasserschutzvorschriften einhalten. Da das Kiesbett nur den Bodenbereich, welcher aufgrund der Ausgestaltung nur einen vergleichsweise geringen Durchmesser aufweist, untermauern muss, ist dessen Herstellung deutlich günstiger als bei herkömmlichen Behältern.

Besonders günstig ist es bei einer bevorzugten Variante der Biogasanlage, wenn auch der verjüngte Abschnitt in das Erdreich eingelassen ist. Der untere Abschnitt des Behälters ist demgemäß nach Art einer Lagune, also eines mit Folie ausgekleideten Erdbeckens, ausgestaltet. Aufgrund des erfindungsgemäß ausgestalteten Bodenabschnitts mit ringförmig geschlossenen Zirkulationskanal können jedoch die Nachteile hinsichtlich Sedimentablagerungsvermeidung, die bei herkömmlichen Lagunen auftreten, mit Vorteil vermieden werden.

Die Erfindung wird in einer bevorzugten Ausführungsform unter Bezugnahme auf eine Zeichnung beispielhaft beschrieben, wobei weitere vorteilhafte Einzelheiten den Figuren der Zeichnung zu entnehmen sind.

Funktionsmäßig gleiche Teile sind dabei mit denselben Bezugszeichen versehen.

Die Figuren der Zeichnung zeigen im Einzelnen:
- Fig. 1:: Vertikalschnitt durch eine Biogasanlage gemäß der Erfindung in einer bevorzugten Ausgestaltungsform mit einem erfindungsgemäßen Behälter in bevorzugter Ausgestaltungsform;
- Fig. 2:: Detailansicht des Bereichs II aus Fig. 1, welche den Bodenbereich der Biogasanlage gemäß Fig. 1 zeigt;
- Fig. 3:: schematische Draufsicht einer bevorzugten Ausgestaltungsform eines Bodenabschnitts eines Behälters nach der Erfindung.

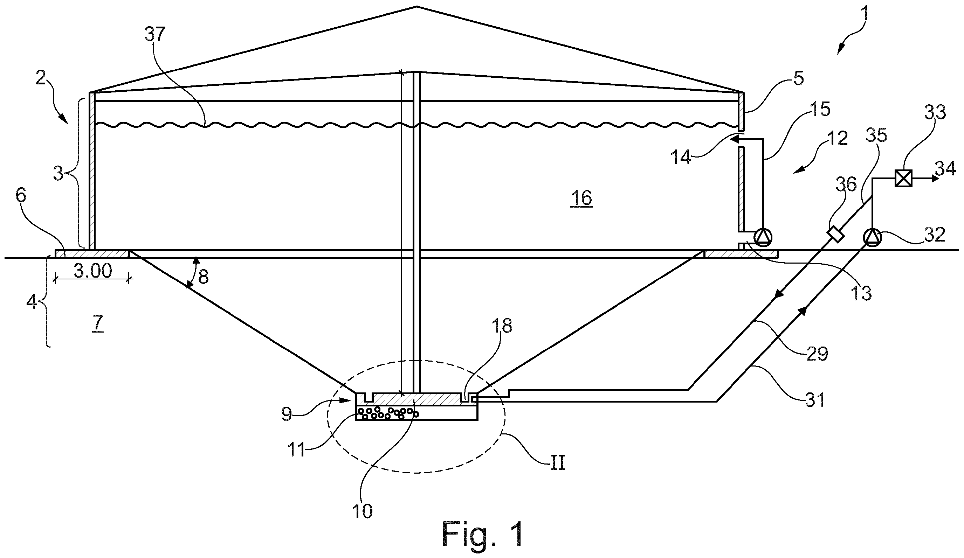

Die Fig. 1 zeigt in einer vertikalen Schnittansicht in bevorzugter Ausgestaltungsform eine Biogasanlage 1 nach der Erfindung mit einem Behälter 2 in einer Ausgestaltungsform der Erfindung. Wie in Fig. 1 zu erkennen, weist der Behälter 2 einen zylindrischen Hauptabschnitt 3 auf, an welchen sich nach unten ein konisch verjüngter Abschnitt 4 anschließt. Der zylindrische Hauptabschnitt 3 weist eine Wandung 5 aus Stahlbeton auf. Die Wandung 5 gründet auf einem ringförmigen Sockelfundament 6.

Der konisch verjüngte Abschnitt 4 befindet sich nach Art einer Lagune im Erdreich 7. Der verjüngte Abschnitt 4 ist aus PE-HD hergestellt. Die Wandung des verjüngten Abschnitts 4 steht in einem Winkel 8 von 10° - 45°, vorzugsweise 30°, zur Horizontalen.

Der verjüngte Abschnitt 4 schließt nach unten mit einem Bodenabschnitt 9 ab. Der Bodenabschnitt 9 besteht im Wesentlichen aus einer Bodenplatte 10 aus Stahlbeton. Die Bodenplatte 10 ist auf einem im Erdreich 7 im Bereich der Bodenplatte 10 vorgesehenen Kiesbett 11 gelagert, um gegebenenfalls Grundwasser abzupumpen.

Der Durchmesser des zylindrischen Hauptabschnitts 3 beträgt gemäß dem hier beschriebenen Ausführungsbeispiel etwa 24 m, wohingegen der Durchmesser der Bodenplatte 10 gemäß dem Beispiel etwa 5 m beträgt. Der konisch verjüngte Abschnitt 4 weist daher eine vertikale Ausdehnung von etwa 6 m auf, entsprechend dem Winkel 8.

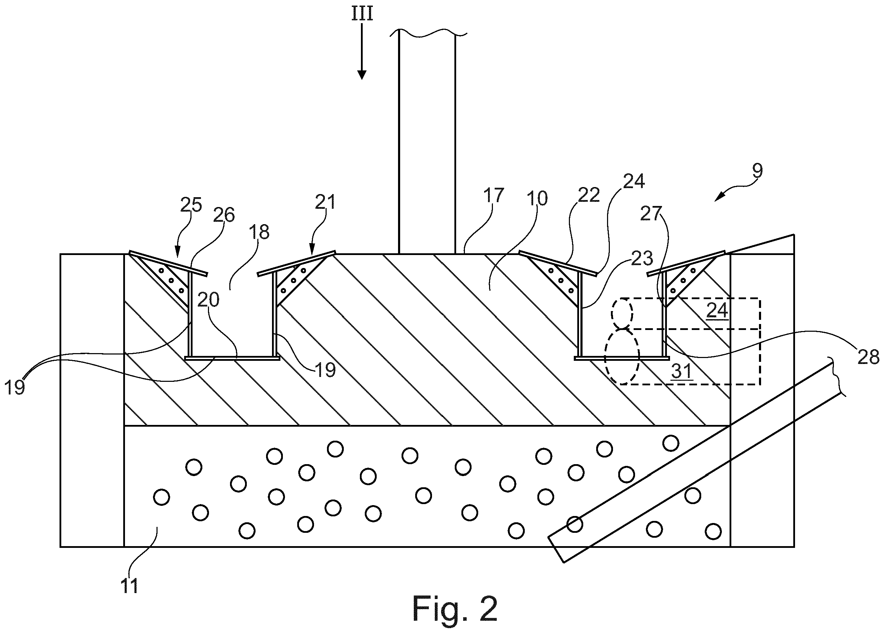

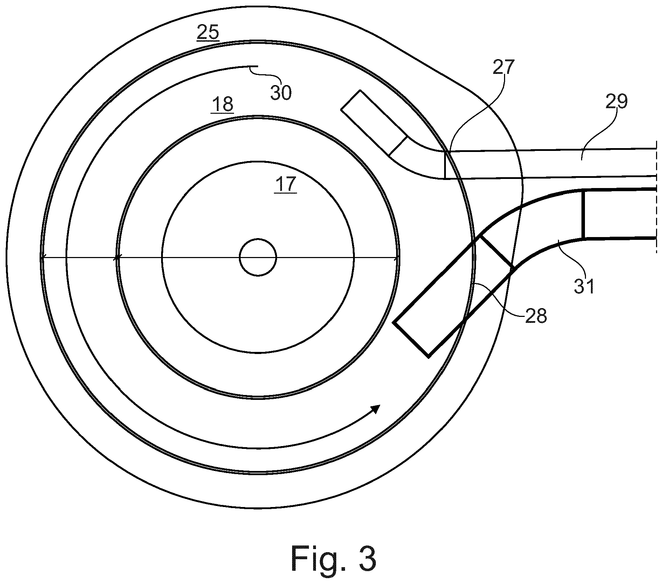

Die Wandung 5 des Hauptabschnitts 3 des erfindungsgemäßen Behälters 2 ist mit einem Rührsystem 12 versehen. Das Rührsystem 12 besteht im Wesentlichen aus einer unteren Öffnung 13 in der Wandung 5 und einer oberen Öffnung 14 in der Wandung 5 sowie eine die untere Öffnung 13 mit der oberen Öffnung 14 verbindenden Rohrleitung 15. In der Rohrleitung 15 ist eine Pumpe angeordnet, um im zylindrischen Hauptabschnitt 3 des Behälters 2 gespeichertes Medium 16 von der unteren Öffnung 13 zu entnehmen und über die obere Öffnung 14 in den Behälter 2 rückzuführen. Auf diese Weise lasst sich eine Umwälzung erzeugen, damit in den Medien 16 enthaltene Nährstoffe möglichst gleichmäßig den aktiven Bakterien zugefügt werden. Nachfolgend wird mit Bezug auf die Figuren 2 und 3 der Bodenabschnitt 9 des Behälters 2 der Biogasanlage 1 gemäß der Fig. 1 näher erläutert. Die Fig. 2 zeigt in diesem Zusammenhang eine Vergrößerung des Ausschnitts II aus Fig. 1. Wie in der Fig. 2 zu erkennen, weist die Bodenplatte 10 aus Stahlbeton einen im Wesentlichen ebenen Zentralbereich 17 auf. Der ebene Zentralbereich 17 ist ringförmig umgeben von einem Ringkanal 18. Der Ringkanal 18 besteht aus Polyethylenplatten 19, welche mit der Bodenplatte 10 aus Stahlbeton vergossen sind. Der Boden 20 des Ringkanals 18 liegt unterhalb des ebenen Zentralbereichs 17 der Bodenplatte 10.

Im Übergang zwischen dem ebenen Zentralbereich 17 der Bodenplatte 10 und dem Ringkanal 18 befindet sich ein gegenüber der Horizontalen geneigter, ringförmiger Übergangsbereich 21. Der geneigte Übergangsbereich 21 ist gemäß dem hier vorgestellten Ausführungsbeispiel mit einer Polyethylenplatte 22 ausgekleidet. Die Polyethylenplatte 22 überragt die senkrechte innere Wandung 23 des Ringkanals 18 am radial äußeren Ende 24 dachartig. Außerdem weist die Bodenplatte 10 im radial äußeren Bereich einen weiteren radial nach innen geneigten Bereich 25 auf, welcher den Ringkanal 18 seinerseits ringförmig umgibt. Der geneigte Außenbereich 25 ist wie der geneigte Übergangsbereich 21 mit Polyethylenplatten 26 verkleidet, welche in den Ringkanal 18 dachartig hineinragen.

Die Fig. 3 zeigt eine Draufsicht auf den Bodenabschnitt 9 in Blickrichtung des Pfeils III in Fig. 2. Wie besonders gut in Fig. 3 zu erkennen, weist der Ringkanal 18 eine Einlassöffnung 27 und eine Auslassöffnung 28 auf. Durch die Einlassöffnung 27 ist ein Einlassrohr 29 aus Polyethylen derart geführt, dass bei der Zufuhr von Medium durch das Einlassrohr 29 in tangentialer Richtung ein Ringstrom 30 in Richtung gegen den Uhrzeigersinn erzeugt wird.

Auf der anderen Seite ist durch die Auslassöffnung 28 ein Auslassrohr 31 geführt, durch welches in tangentialer Richtung Medium aus dem Ringkanal 18 entnommen werden kann, um den Ringstrom 30 zu erzeugen bzw. zu verstärken.

Nochmals zurückkommend auf Fig. 1 wird nachstehend die Wirkung des Einlassrohrs 29 und des Auslassrohrs 31 zur Erzeugung des Ringstroms 30 im Ringkanal 18 näher erläutert. Wie in Fig. 1 zu erkennen, ist das Auslassrohr 31 an eine Pumpe 32 angeschlossen, welche über ein Ventil 33 mit einem nicht näher dargestellten Lagerbehälter 34 kommuniziert. Weiter ist ersichtlich, dass am Ausgang der Pumpe 32 eine Bypass-Leitung 35 vorgesehen ist, welche das Auslassrohr 31 mit dem Einlassrohr 29 zusammenschließt. Weiter ist zu erkennen, dass in der Bypass-Leitung 35 ein nur schematisch dargestellter Schieber 36 vorhanden ist. Mithilfe des Schiebers 36 lässt sich das Verhältnis des von der Pumpe 32 geförderten Mediums, welches über die Bypassleitung 35 und das Einlassrohr 29 durch die Einlassöffnung 27 in den Ringkanal 18 rückgeführt wird, zudem Anteil Medium, welcher in den Lagerbehälter 34 geführt wird, einstellen.

Im Übrigen sind in Fig. 1 weitere Komponenten der Biogasanlage 1, etwa zur Entnahme des erzeugten Biogases, nicht näher dargestellt. Diese können in herkömmlicher, dem zuständigen Fachmann wohlbekannter Weise ausgestaltet sein.

Zum Betrieb der Biogasanlage 1 mit dem Behälter 2 gemäß den Figuren 1 bis 3 nach der Erfindung wird über eine in Fig. 1 nicht dargestellte Öffnung Medium 16 in den Behälter 2 bis zu einem Füllstand 37 eingefüllt. Das Medium 16 ist Biomasse als Ausgangsstoff für die Biogaserzeugung. Als Medium 16 kommen auch Schlämme mit hohem Schwerstoffanteil/Sinkstoffanteil etwa aus Sand oder Grit in Betracht. Um sicherzustellen, dass insbesondere bei großen Durchmessern des Hauptabschnitts 3, beispielsweise im Bereich von 10-40 m, die Bakterien mit organischen Nährstoffen versorgt und in der Schwebe gehalten werden, dient zunächst das Rührsystem 12, welches eine Umwälzung des Mediums 16 im Behälter 2 sicherstellt.

Gleichzeitig dient jedoch erfindungsgemäß der Ringstrom 30 im Ringkanal 18 in der Bodenplatte 10 dazu, eine Verkrustung und Trichterbildung im Bodenabschnitt 9 des Behälters 2 zu unterbinden. Dazu wird mittels der Pumpe 32 über das Auslassrohr 31 und die Auslassöffnung 28 im Ringkanal 18 Schlamm abgesaugt, welcher über das Ventil 33 einem Lagerbehälter zugeführt werden kann. Um jedoch eine Ringströmung 30 im Ringkanal 18 aufrechtzuerhalten, wird über die Bypass-Leitung 35 und das Einlassrohr 29 durch die Einlassöffnung 27 ein Teil des über das Auslassrohr 31 abgesaugten Schlamms in den Ringkanal 18 rückgeführt. Dabei kann das Verhältnis des Rückführungsstroms über den nicht näher dargestellten Schieber 36 in der Bypass-Leitung 35 eingestellt werden.

Die Ausgestaltung des Schiebers 36 kann in jeder beliebigen, dem Fachmann bekannten Weise sein, beispielsweise als Regelventil.

Aufgrund der Ringströmung 30 im Ringkanal 18 wird mit vergleichsweise geringem Energieeinsatz eine Vermeidung von Ablagerungen von Sinkstoffen im Bodenabschnitt 9 des Behälters 2 bewirkt. Daher kann eine Umwälzung anhand des Rührsystems 12 im zylindrischen Hauptabschnitt 3 des Behälters 2 mit vergleichsweise geringem Energieaufwand betrieben werden, ohne dass Probleme auftreten.

Dadurch, dass zwischen dem zylindrischen Hauptabschnitt 3 mit großem Durchmesser, beispielsweise 15 m oder mehr, und dem Bodenabschnitt 9 der konisch verjüngte Abschnitt 4 mit Polyethylenwandungen angeordnet ist, weist der Bodenabschnitt 9 einen erheblich geringeren Durchmesser, beispielsweise 3 m, auf als der Hauptabschnitt 3.

Bezüglich der Einstellung des über die Bypassleitung 35 durch das Einlassrohr 29 rückgeführten Anteils an Medium ist nach der Erfindung eine Voreinstellung gegeben, indem der Durchmesser des Einlassrohrs 29 kleiner gewählt ist als der Durchmesser des Auslassrohrs 31. Als besonders günstig hat es sich erwiesen, wenn der Durchmesser des Einlassrohrs 29 110 mm beträgt und der Durchmesser des Auslassrohrs 31 etwa 200 mm beträgt.

Auf diese Weise sind ein Behälter und ein Biogasanlage offenbart, welche mit geringeren Investitionskosten und geringeren Betriebskosten als herkömmliche Anlagen und Behälter betrieben werden können, ohne dass Verstopfungen und Sedimentierungen aufgrund von Sinkstoffen auftreten.

### BEZUGSZEICHENLISTE

- 1: Biogasanlage
- 2: Behälter
- 3: Hauptabschnitt
- 4: konisch verjüngter Abschnitt
- 5: Wandung
- 6: Sockelfundament
- 7: Erdreich
- 8: Winkel
- 9: Bodenabschnitt
- 10: Bodenplatte
- 11: Kiesbett
- 12: Rührsystem
- 13: untere Öffnung
- 14: obere Öffnung
- 15: Rohrleitung
- 16: Medium
- 17: ebener Zentralbereich
- 18: Ringkanal
- 19: PE-Platten
- 20: Boden
- 21: geneigter Übergangsbereich
- 22: PE-Platte
- 23: innere Wandung
- 24: äußeres Ende
- 25: geneigter Außenbereich
- 26: PE-Platte
- 27: Einlassöffnung
- 28: Auslassöffnung
- 29: Einlassrohr
- 30: Ringstrom
- 31: Auslassrohr
- 32: Pumpe
- 33: Ventil
- 34: Lagerbehälter
- 35: Bypassleitung
- 36: Schieber
- 37: Füllstand

## Patentansprüche

1. Behälter (2), insbesondere zur Erzeugung von Biogas, mit einem, vorzugsweise zylindrischen, Hauptabschnitt (3) und einem unterhalb des Hauptabschnitts (3) angeordneten Bodenabschnitt (9), **dadurch gekennzeichnet, dass** der Bodenabschnitt (9) einen ringförmig geschlossenen Zirkulationskanal (18) aufweist, wobei der Zirkulationskanal (18) eine Einlassöffnung (27, 29) mit einem Einlassquerschnitt aufweist, um im wesentlichen tangential zur Hochachse des Behälters (2) Medium in den Zirkulationskanal (18) einzulassen, wobei der Zirkulationskanal (18) eine Auslassöffnung (28, 31) mit einem Auslassquerschnitt aufweist, um im wesentlichen tangential zur Hochachse des Behälters (2) Medium aus dem Zirkulationskanal (18) abzuführen, wobei eine die Auslassöffnung (28, 31) mit der Einlassöffnung (27, 29) verbindende Bypassleitung (35) vorgesehen ist, wobei Mittel (36) zum Variieren eines Querschnitts der Bypassleitung (35) vorgesehen sind

2. Behälter (2) nach Anspruch 1, **dadurch gekennzeichnet, dass** der Bodenabschnitt (9) einen im wesentlichen ebenen Zentralbereich (17) aufweist, welcher horizontal von dem Zirkulationskanal (18) begrenzt ist.

3. Behälter (2) nach einem der vorhergehenden Ansprüche, **dadurch gekennzeichnet, dass** eine Einlasspumpe (32) zum Einlassen von Medium in den Zirkulationskanal (18) über die Einlassöffnung (27, 29) und/oder eine Auslasspumpe (32) zum Abführen von Medium aus dem Zirkulationskanal (18) über die Auslassöffnung (28, 31) vorgesehen ist.

4. Behälter (2) nach einem der vorhergehenden Ansprüche, **dadurch gekennzeichnet, dass** der Auslassquerschnitt größer als der Einlassquerschnitt ausgestaltet ist.

5. Behälter (2) nach einem der vorhergehenden Ansprüche, **dadurch gekennzeichnet, dass** der Zirkulationskanal (18) aus einem Kunststoff hergestellt ist und vorzugsweise mit dem Zentralbereich (17) vergossen ist.

6. Behälter (2) nach einem der vorhergehenden Ansprüche, **dadurch gekennzeichnet, dass** sich in Richtung der Hochachse des Behälters (2) an den Bodenabschnitt ein in Richtung zum Bodenabschnitt, vorzugsweise konisch, verjüngter Abschnitt (4) anschließt.

7. Behälter (2) nach einem der vorhergehenden Ansprüche, **dadurch gekennzeichnet, dass** der verjüngte Abschnitt (4) Kunststoffwandungen aufweist.

8. Behälter (2) nach einem der vorhergehenden Ansprüche, **dadurch gekennzeichnet, dass** im Hauptabschnitt Rührmittel (12, 13, 14, 15) zum Rühren im Behälter (2) befindlichen Mediums (16) vorgesehen sind.

9. Biogasanlage (1), **dadurch gekennzeichnet, dass** sie einen Behälter (2) nach einem der Ansprüche 1 bis 8 aufweist, der zumindest mit dem Bodenabschnitt (9) in das Erdreich (7) eingelassen ist, wobei unterhalb des Bodenabschnitts (9) ein Kiesbett (11) vorgesehen ist.

10. Biogasanlage (1) nach Anspruch 9, **dadurch gekennzeichnet, dass** auch der verjüngte Abschnitt (4) in das Erdreich (7) eingelassen ist.

## Claims

1. Container (2), in particular for the production of biogas, comprising a preferably cylindrical main portion (3) and a base portion (9) arranged below the main portion (3), **characterised in that** the base portion (9) has an annularly closed circulation channel (18), the circulation channel (18) having an inlet opening (27, 29) with an inlet cross section in order to admit medium into the circulation channel (18) substantially tangentially to the vertical axis of the container (2), the circulation channel (18) having an outlet opening (28, 31) with an outlet cross section in order to discharge medium out of the circulation channel (18) substantially tangentially to the vertical axis of the container (2), a bypass line (35) being provided that connects the outlet opening (28, 31) to the inlet opening (27, 29), and means (36) being provided for varying a cross section of the bypass line (35).

2. Container (2) according to claim 1, **characterised in that** the base portion (9) has a substantially planar central region (17) which is delimited horizontally by the circulation channel (18).

3. Container (2) according to either of the preceding claims, **characterised in that** an inlet pump (32) is provided for admitting medium into the circulation channel (18) via the inlet opening (27, 29) and/or an outlet pump (32) is provided for discharging medium out of the circulation channel (18) via the outlet opening (28, 31).

4. Container (2) according to any of the preceding claims, **characterised in that** the outlet cross section is designed to be larger than the inlet cross section.

5. Container (2) according to any of the preceding claims, **characterised in that** the circulation channel (18) is made from a plastics material and is preferably cast together with the central region (17).

6. Container (2) according to any of the preceding claims, **characterised in that** a portion (4) which tapers, preferably conically, in the direction of the base portion adjoins the base portion in the direction of the vertical axis of the container (2).

7. Container (2) according to any of the preceding claims, **characterised in that** the tapered portion (4) has plastics walls.

8. Container (2) according to any of the preceding claims, **characterised in that** stirring means (12, 13, 14, 15) for stirring medium (16) located in the container (2) are provided in the main portion.

9. Biogas plant (1), **characterised in that** it comprises a container (2) according to any of claims 1 to 8, at least the base portion (9) of which is embedded into the ground (7), with a gravel bed (11) being provided beneath the base portion (9).

10. Biogas plant (1) according to claim 9, **characterised in that** the tapered portion (4) is also embedded into the ground (7).

## Revendications

1. Contenant (2), en particulier servant à produire du biogaz, avec une section principale (3), de préférence cylindrique, et une section de fond (9) disposée sous la section principale (3), **caractérisé en ce que** la section de fond (9) présente un canal de circulation (18) fermé de manière annulaire, dans lequel le canal de circulation (18) présente une ouverture d'entrée (27, 29) avec une section transversale d'entrée pour faire entrer un milieu dans le canal de circulation (18) sensiblement de manière tangentielle par rapport à l'axe en hauteur du contenant (2), dans lequel le canal de circulation (18) présente une ouverture de sortie (28, 31) avec une section transversale de sortie pour évacuer un milieu hors du canal de circulation (18) sensiblement de manière tangentielle par rapport à l'axe en hauteur du contenant (2), dans lequel un conduit de dérivation (35) reliant l'ouverture de sortie (28, 31) à l'ouverture d'entrée (27, 29) est prévu, dans lequel des moyens (36) servant à faire varier une section transversale du conduit de dérivation (35) sont prévus.

2. Contenant (2) selon la revendication 1, **caractérisé en ce que** la section de fond (9) présente une zone centrale (17) sensiblement plane, laquelle est délimitée horizontalement par le canal de circulation (18).

3. Contenant (2) selon l'une quelconque des revendications précédentes, **caractérisé en ce que** sont prévues une pompe d'entrée (32) pour faire entrer un milieu dans le canal de circulation (18) par l'intermédiaire de l'ouverture d'entrée (27, 29) et/ou une pompe de sortie (32) servant à évacuer un milieu hors du canal de circulation (18) par l'intermédiaire de l'ouverture de sortie (28, 31).

4. Contenant (2) selon l'une quelconque des revendications précédentes, **caractérisé en ce que** la section transversale de sortie est configurée de manière plus grande que la section transversale d'entrée.

5. Contenant (2) selon l'une quelconque des revendications précédentes, **caractérisé en ce que** le canal de circulation (18) est fabriqué à partir d'une matière plastique et est scellé de préférence à la zone centrale (17).

6. Contenant (2) selon l'une quelconque des revendications précédentes, **caractérisé en ce qu'**une section rétrécie (4) de préférence de manière conique en direction de la section de fond se raccorde à la section de fond en direction de l'axe en hauteur du contenant (2).

7. Contenant (2) selon l'une quelconque des revendications précédentes, **caractérisé en ce que** la section rétrécie (4) présente des parois en matière plastique.

8. Contenant (2) selon l'une quelconque des revendications précédentes, **caractérisé en ce que** des moyens d'agitation (12, 13, 14, 15) servant à agiter un milieu (16) se trouvant dans le contenant (2) sont prévus dans la section principale.

9. Installation de biogaz (1), **caractérisée en ce qu'**elle présente un contenant (2) selon l'une quelconque des revendications 1 à 8, qui est enterré dans le sol (7) au moins par la section de fond (9), dans laquelle un lit de gravier (11) est prévu sous la section de fond (9).

10. Installation de biogaz (1) selon la revendication 9, **caractérisée en ce que** la section rétrécie (4) est également enterrée dans le sol (7).
